(19) Europäisches Patentamt
European Patent Office
Office européen des brevets

(11) **EP 2 098 970 A1**

## (12) EUROPEAN PATENT APPLICATION

(43) Date of publication:
**09.09.2009 Bulletin 2009/37**

(51) Int Cl.:
**G06F 19/00** (2006.01)

(21) Application number: **08250773.2**

(22) Date of filing: **07.03.2008**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(71) Applicant: **British Telecommunications public
limited company
81 Newgate Street
EC1A 7AJ (GB)**

(72) Inventor: **The designation of the inventor has not
yet been filed**

(74) Representative: **Williamson, Simeon Paul et al
BT Group Legal
Intellectual Property Department
PP C5A
BT Centre
81 Newgate Street
London EC1A 7AJ (GB)**

## (54) Abnormal event time thresholds

(57)    Apparatus for alerting an operator to the status of a monitored system, the apparatus comprising:
- input means for permitting an operator to input utility values associated by the operator with different courses of action and different states of the system being monitored, such that each utility value expresses the perceived value to the operator of carrying out different courses of action when the monitored system is in each of a plurality of different states;
- monitoring means for estimating the probability of the monitored system being in each of a plurality of different states based on one or more sensed parameters;
- expected utility calculation means for calculating an expected utility for each possible course of action based on the utility values associated with the different courses of action and the estimated likelihoods of the different possible states of the monitored system; and
- alerting means for alerting the operator if a predetermined condition based on the calculated expected utilities is satisfied.

FIGURE 2

**Description**

This invention relates to apparatus, systems and methods for the selection and setting of threshold values in remote status monitoring, particularly but not exclusively in the context of the remote monitoring of the wellbeing of persons.

"Telecare" is a term describing the use of technology to enable parties such as care providers (CPs) to monitor the status or wellbeing of persons who may be elderly or otherwise vulnerable (referred herein as customers or service users (SUs)), where such SUs remain in their own homes or are otherwise located remote to the CPs.

Various telecare approaches are known. In the most direct method of obtaining SU data, SUs wear devices which measure certain physical or physiological parameters. For example, accelerometer-based equipment worn on the person can provide direct feedback to the CP or other party administrating or operating the telecare system, that the SU has fallen. However this method suffers from being excessively invasive to the SU who may have to have on his person a number of such devices. Compliance is also a problem where the SU is reluctant to cooperate by wearing the device(s) especially if interaction with a complex user interface is required. High cost of implementation is yet another deterrent to the widespread adoption of such solutions.

Another approach is to provide ambient sensors fixed within the SU's premises, to monitor the SU's activities. This can be achieved by use of devices which literally keep an eye on the SU, such as video cameras and sound recording devices. However this approach could also be ethically objectionable for being excessively invasive; it is also expensive and complicated to set up and monitor.

A third and preferred approach is to capture data indirectly about the SU's movements and actions. Such data is obtained by use of sensors such as passive infrared (PIR) motion sensors, sensors to detect door and window opening and closure, meters to detect use of water, electric and gas, and the like - which provide information about the SU's activities within the premises in a relatively non-invasive way. These devices can capture information about the activity and inactivity of the SU with greater subtlety than by use of wearable devices or by video cameras, and being technologically and commercially mature technologies, are relatively reliable and inexpensive to obtain, install (especially if they do not require wiring) and use. Sensed atypical inactivity, in particular, can be mapped to and signify an abnormal event, such as a fall. If the system senses and interprets that such an abnormal event has indeed occurred, an alarm can be raised to the remote CPs. In this description, the term "event", depending on context, includes the occurrence of a event (sometimes "positive event") as well as absence of events (a "negative event" or "non-event"), and the term "time interval" or the like may relate to the time expired between such events and non-events.

The present invention has application in any remote monitoring system using any approach i.e. regardless of how the data is obtained. However an exemplary embodiment will be described herein in the main in the context of a telecare system based on a motion sensor-based system. It is also applicable regardless of how the timing and other data is obtained, although the description herein will in the main refer to an embodiment and application in the context of a system which gathers data through sensor nodes or devices which sense physically-discernable changes in the environment of the SU, such as motion sensors, door and window opening and closing sensors, bed occupancy, toilet usage, utility use meters and the like.

In such a system, the gathered information is analysed to determine if a pre-determined condition is met. As an example, periods of non-movement can be identified by the system to denote an abnormality so that a period of non-movement exceeding a pre-determined length of time is deemed to be abnormal or unusual, and indicative that the SU has fallen within the premises or is otherwise incapacitated.

In the present description, an "inter-event" time period is the length of time elapsed between consecutive positive events detected by either the same sensor or all sensors within the dwelling of a particular SU. Put another way, an "inter-event" period is the duration of a negative event. In the monitoring or telecare system, a threshold value defines the boundary of an acceptable, or "normal", inter-event period. If the sensor, or group of sensors, fails to detect an event beyond a set threshold value, this may be deemed to be an abnormal occurrence deserving attention. Thus, the term "inter-event time interval" could also in an appropriate context refer to a time interval following the end of a positive event where there may be a late or even no following positive event, especially in the context of discovering if any positive event ending this time period occurs soon enough to be acceptable.

One major problem for the telecare system operator or administrator is to decide how, and where, to set the threshold value of the non-movement period or time interval. If the value is set too low, then an excessive number of false alarms will be generated, annoying all concerned, incurring unnecessary cost, and reducing trust in the system. Setting the threshold level too high however, carries the risk of an alarm being raised late which means that assistance would be sent (too) late to the SU needing help.

One solution to the determination of the threshold value is to simply set an arbitrary fixed value. For example, an alarm is raised if no activity is detected within a fixed period e.g. within eight hours. In some implementations, this value may be subsequently re-set. This is currently the prevalent method deployed by telecare operators, where sensors (also here referred to as nodes) have their threshold values either factory-set, or set by the engineer installing the sensors at the premises or dwellings. Such a "one-size-fits-all" approach is seldom satisfactory, as SUs differ widely in overall activity levels and habit. The same one SU's activities can also vary at different times of the day, week or year. Similarly, different rooms see different levels of activity - again, this varies on the time of the day or the year. The relationship between the set value and the telecare policy adopted by the CP is also not taken into account. The threshold value is thus often at best an educated guess based perhaps historical data about its applicability or accuracy in the particular implementation. As may be expected, such assumptions and historical data may have little or no application in the immediate case.

As an alternative to fixed threshold values, "adaptive" threshold setting strategies, which are personalised to the particular sensor node for a certain time of day, have been developed. Setting the thresholds always involves a trade-off between the *sensitivity* of the system (its ability to detect conditions of concern) and its *specificity* (the ability of the system to not raise an alert when there is no cause for concern). Therefore one obvious method of settings thresholds is to set an acceptable maximum for the number of alerts that are generated in a specific time period (say, an average one alert per node per month) and setting the alert threshold to the value that results in that number of false alerts. This approach suffers from a number of problems. First, the threshold value is determined entirely for the administrative convenience of the provider, with no consideration of the needs of the service user. There is no consideration of the consequences (social, medical or financial) of the failure the system to detect a condition of concern or the consequences (financial) of sending a response team. A decision-making process should preferably take into account such consequences. Use of a system based on such a decision-making process may give rise to uneven results where the standard of care given to SUs (or where appropriate, a segment or class thereof) is not the same.

Another way is to set thresholds which are a certain number (say, three) standard deviations from the mean time between sensed events, which is a measure of how "unusual" a particular time value is. These approaches are to some extent tailored to individual SUs, but the criteria generating the adaptive threshold values are still are set according to subjective criteria by the factory, the installer, or the CP, and not empirical data based on actual SU activity.

The adaptive approach is also discussed in Barnes et al. "Case Studies from the Liverpool Telecare Pilot" (Informatics in Primary Care 2006 14(3):197-202), which describes a project involving the applicants. It is mentioned in these Case Studies that the threshold value is obtained by use of an adaptive algorithm which establishes the thresholds using Bayesian decision theory. Aspects of a telecare system and how a threshold can be established using an Adaptive Threshold Algorithm (ATA), which are based on the applicant's experience in the Liverpool Telecare Pilot, are described on co-pending application no. EP 07253336.7, which is described more fully below.

There is therefore a need for a way to set a personalised time threshold value to indicate when a care giver or the telecare administrator should take action based on the probability that the particular monitored SU is in need of help.

At the same time, there is also the need for a solution which addresses issues around the design and implementation of a telecare system from the CP's perspective, which looks at the trade-off between the generation of too many false positive and false negatives, between taking action when not actually required, and sending help (too) late.

The financial, social and other costs and risks associated with wrongly taking or desisting from action are major considerations in the setting up and implementation of such telecare systems. Co-existing with the day-to-day running of the system, there is also a need for clear and transparent accountability especially in the context of publicly-funded and -run healthcare initiatives. This aspect particularly concerns issues of efficacy of the system, efficiency of use, and the equal availability and fairness of care of all within the SU base. Absence of mechanisms allowing for the review of these factors in the implementation and running of the system - including the setting of time thresholds - is an obstacle to the large-scale or commercial deployment of such a telecare system, especially where the initiative is funded by the public purse.

Threshold-setting is a key aspect of the operation of the system, as it can cause either an alarm to be triggered and action to be action, or else to continue inaction. Such situations involve a choice to be made between options carrying a cost/benefit trade-off. CP entities with finite resources need to, as an administrative decision, decide how best to maximise utility or benefit according to the policy it has chosen to adopt.

In a first aspect of the invention, there is provided apparatus for alerting an operator to the status of a monitored system, the apparatus comprising:

- input means for permitting an operator to input utility values associated by the operator with different courses of action and different states of the system being monitored, such that each utility value expresses the perceived value to the operator of carrying out different courses of action when the monitored system is in each of a plurality of different states;
- monitoring means for estimating the likelihood of the monitored system being in each of a plurality of different states based on sensed data;
- expected utility calculation means for calculating an expected utility for each possible course of action based on the utility values associated with the different courses of action and the estimated likelihoods of the different possible states of the monitored system; and

- alerting means for alerting the operator if a predetermined condition based on the calculated expected utilities is satisfied.

According to the invention, a monitored system can take a number of states, to which an operator may respond in a variety of ways. The apparatus takes into account the utility value of various responses which the CP may make, based on the likelihood or probability of existence of the state of the system. Within the telecare context, the apparatus can be used to allow a CP to determine the expected utility value of responding in a certain way to a likely SU state, e.g. where the SU needs help, or where the SU does not need help. In preferred implementations, the likelihood factor is generated by statistically generating a metric such as the shape parameter and a scale parameter when fitting the data sensed from the sensing nodes, which characterise the sensed data. A forecaster can also advantageously included in the apparatus which will generate an expected number of alerts or alarms that may be raised if the CP take a particular course of action in response to a likely state of affairs. This helps in the planning and setting up of the monitoring apparatus and system.

In a second aspect of the invention, there is provided a remote monitoring system including

- apparatus according to the invention, and
- a node for collecting the sensed data.

In a third aspect of the invention, there is provided a telecare monitoring system including the remote monitoring apparatus of the invention.

In a further aspect of the invention, there is provided a method for alerting an operator to the status of a monitored system, the apparatus comprising:

- an operator inputting utility values associated by the operator with different courses of action and different states of the system being monitored, such that each utility value expresses the perceived value to the operator of carrying out different courses of action when the monitored system is in each of a plurality of different states;
- estimating the likelihood of the monitored system being in each of a plurality of different states based on sensed data;
- calculating an expected utility for each possible course of action based on the utility values associated with the different courses of action and the estimated likelihoods of the different possible states of the monitored system; and
- alerting the operator if a predetermined condition based on the calculated expected utilities is satisfied.

Methods directed to the operation of a remote monitoring, and separately, the operation of a telecare system using the method of the invention are also provided.

Embodiments of the invention will now be described by way of example only with reference to the accompanying drawings in which:

Figure 1 depicts the components of a telecare system set up to generate and use adaptive threshold values;
Figure 2 depicts the components of a telecare system set up to generate and use adaptive threshold values in accordance with a telecare policy adopted by the care provider;
Figure 3 depicts process sequence flows in an exemplary embodiment of the invention;
Figure 4 is another view of the process flows;
Figure 5 is a graph showing sensed data fitted to a probability distribution; and
Figure 6 is a graphical depiction of the expected utility levels for two care provider responses.

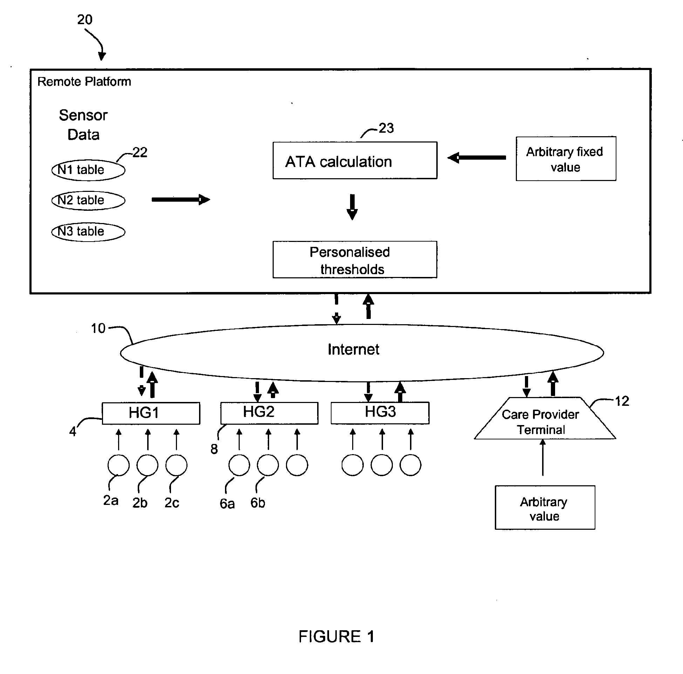

Figure 1 depicts an architecture for a telecare system suitable for the generation of ATA-derived threshold values, as described in parts of EP 07253336.7. Essentially, a number of nodes (2a, 2b, 2c) positioned within the dwelling of an SU which may take the form of PIR sensors, meter sensors and the like. Sensed motion events and the like indicating SU activity are transmitted to the home gateway (4). A home gateway typically functions as the central residential monitoring unit of the premises, to which each node communicates its sensed data. These signals are then transmitted via a wider network such as the Internet (10) to a remote platform (20). As can be seen, nodes (6a, 6b, etc.) in a different SU residence or premises is arranged to send their sensed data to the remote platform via another home gateway (8) located in the premises of the other SU. The remote gateway compiles the sensed data from a number of premises and stores them in the form of tables (22).

In the ATA approach, an ATA calculator (23) generates a time threshold value for a node for a particular time of day, by calculating an abnormality statistical measure from a number of events sensed by that node, e.g. by reference to a specific number of standard deviations from the mean in the range of sensed data. A personalised threshold value for that node for the relevant time of day is generated, and then sent back to the home gateway of each of the premises, which stores the value and any subsequent updates to the value. Using this value, the home gateway can, as a matter of probability, determine if a time interval reported by the particular node should be regarded as an abnormality requiring an alarm to be generated.

Typically however, ATA-derived values are obtained from criteria set by the CP based on arbitrary choice (e.g. an observed time interval that is certain number of standard deviations above the mean and thus not based on any evidence of its pertinence to the particular node at that particular time of day) or on assumptions that may or may not be valid. In such cases, any basis for the choice of the values selected is opaque to a third party, and the implications and consequences of the chosen threshold value may not be visible to even the CP itself.

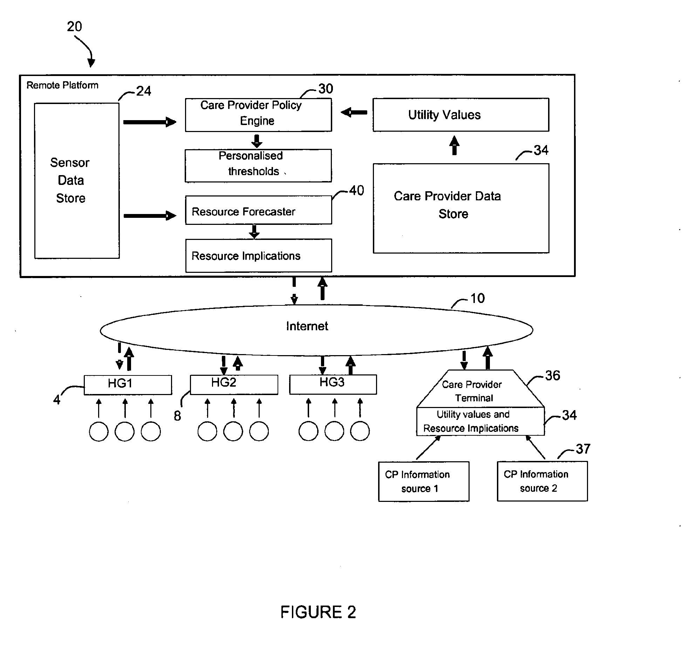

In the exemplary embodiment of the invention shown in Figure 2, the telecare architecture is substantially similar to the system described against Figure 1 above. However, it differs most significantly in the inclusion of a care provider policy engine (CPPE) (30) and a resource forecaster (40).

Specifically, the ATA calculator (23) of the system shown in Figure 1 is replaced with the CPPE (30), which performs the calculation of the threshold value. Similar to the ATA calculator of the system discussed above against Figure 1, the CPPE generates an abnormality statistical measure to produce a personalised lack-of-activity threshold time value for each node for a particular time of day. As previously stated, this indicates that a certain length of time has passed without detection of activity of an SU by a particular node, such that there is a strong likelihood that that SU will need assistance.

In contrast to, and as a refinement on, the ATA approach, the threshold value obtained by the CPPE is obtained according to the telecare policy adopted by the CP. As noted earlier, the telecare policy is based on the CP's overall assessment of the possible outcomes from adoption of the threshold value and appreciation of the risk deemed acceptable for each possible outcome. This empirical approach contrasts with the use of arbitrary values (e.g. "three standard deviations") or historical data which may not be pertinent to the particular node for the particular time of day. This policy is applied to each node within the system for which the CP has responsibility.

In the system architecture shown in Figure 2, sensed data is again sent by the home gateways (4, 8, etc.) and then to the remote platform (20) controlled by the CP. The inter-event time, which is in this discussion denoted by $\tau$ (tau), is obtained from the time elapsed between sensed positive events.

In determining node threshold values, the CP is in practice faced with a number of scenarios and choices for response. In the following example, the CP can classify the status of an SU ($H$) at any given time to be one of two states:

$H_0$ : The SU does not need help or attention
$H_1$ : The SU needs help or attention

In response, the CP has the following response ($D$) choices:

$D_0$ : Do nothing; take no action
$D_1$ : Do something; initiate a response activity

It is convenient to measure utilities on a scale from 0, representing the worst outcome, to 1, representing the best outcome. In this example therefore, there are four combinations of SU status and the provider's responses:

Table 1 State-response combinations

| $H_0 D_0$ | $H_0 D_1$ |
|---|---|
| $H_1 D_0$ | $H_1 D_1$ |

A "cost" is associated with each status/response combination. This cost is widely defined and encompasses all forms of loss: opportunity cost, time, and money - e.g. the financial and opportunity cost incurred in the $H_0 D_1$ combination where the CP sends an ambulance to an SU who is actually fine. The cost can also be expressed in social or other terms in the $H_1 D_0$ combination where the SU does needs help, but the CP fails to take action in time or at all. Put in the converse, there is a utility value which can be attributed to the four state-response combinations for any inter-event time $\tau$ during the operation of the node, the utility being the benefit obtained from incurring the cost associated with the CP response.

As the skilled person may appreciate, it is possible for more than one type of "Do something" response to exist. For example, there may be different reactions based on e.g. level of urgency, such as "Call the SU" as a first response, "Call the SU's relatives as a second response", "Send an ambulance", and so on.

Utility theory is a well known concept wherein the benefit in a particular outcome is measured in terms of its relative satisfaction to a particular party. Thus, the utility for the same outcome may differ for different parties, as it is specific to the context of the one particular party.

By referring to the utility values of each state-response combination, a CP can make rational decisions about how it would best to respond in a particular case, given the circumstances and context it is operating in. An entity with limitless resources may set up its telecare system to unintelligently and extravagantly respond to all four state-response combinations by sending help to the SU, while another CP with finite resources would want to arrange its telecare system to maximise its benefit or utility for not just a particular node at a particular time, but also over the entire telecare system or network, for a given time period.

Referring back to Table 1, a utility value of 0.0 can be attributed to the $H_0 D_1$ combination (where the CP takes unnecessary action), while a value of 1.0 may be attributed to the $H_1 D_1$ combination (where the CP takes responsive action when required). Each state-response combination would then have associated with it a utility value ($U$).

Table 2 State-response utility values

| State-response combinations | $D_0$ | $D_1$ |
|---|---|---|
| $H_0$ | $U_0$ | $U_1$ |
| $H_1$ | $U_2$ | $U_3$ |

The utility values reflect the emphasis given by the CP to each state-response combination. For example, a CP may adopt a policy that it should err on the side of caution, and so choose to send out help in cases even when it is unclear if the SU status is $H_0$ or $H_1$. In such a case, the utility values $U$ can be adjusted to reflect the policy adopted by the CP. Use of utility theory in this way gives to the CP a clear picture of the implications of the choice of policy adopted.

Using the above utility values, it is possible to calculate the expected utility ($EU$) of a selected response. As the inter-event time $\tau$ increases, a point will be eventually reached when the expected utility value of responding will be the same as the expected utility value of not responding. According to the invention, the CP may choose this point as a threshold value or level, as the determining point in time when intervening action should be taken on the basis that it is more probable than not that the SU needs assistance.

The approach in this invention makes use of two key concepts: the expected value of a random variable and the concept of utility. The notion of expected value requires that all possible outcomes and their respective probabilities of occurring be taken into account when making decisions under conditions of uncertainty. By computing expected utility, a value can be put on a situation that has uncertain outcomes with known probabilities. Use of the concept of expected utility allows the comparison of any two situations, irrespective of whether their outcomes are uncertain or entirely risk-free.

The threshold value determined by the above method is calculated for each node for a particular time period (e.g. a node located in a kitchen of a particular SU, for two-hour period from 00:00 to 02:00). The system similarly calculates a set of threshold values for each node for all time periods, which is then sent to, and stored at, the home gateway (4, 6, etc.) of the premises where that particular node is located. The system is capable of being set up so that other parameters are taken into account e.g. there could be further sets of values which reflect the different seasons of the year, and so on.

By adopting threshold values set in this manner for all nodes in all premises, the CP can be seen to be applying the same policy across its SU base in an equitable, non-discriminatory basis to provide the same standard of care to all within its responsibility. Preferably, this tool could also be used to set different standards and levels of alarm sensitivity within the SU network if this is desired, as described further below.

In the exemplary implementation shown in Figure 2, the utility values required by the CPPE to generate the threshold value, are derived from a source of factors (37, 39) which influence the utility values. These factors could include the CP's budget, the "quality of service" standard (e.g. in an implementation administered by a private CP where SUs are subscribed to different tiers or levels of service), and so on.

In one embodiment, care provider data can be just one type of input - or one of many inputs - fed into the CPPE for generation of the threshold value. The system is thus arranged to use utility values which can originate from a variety of sources, either in combination with one or more other sources of utility value determining factors.

Although the system can be set up so that the CPPE processes fixed or static utility values (e.g. in situations wherein such values are not expected to change), the utility values can advantageously comprises relative values. They can also themselves comprise functions which are themselves determined by one or more other factors, which reflects the reality in this complex area where numerous considerations have to be taken into account in deciding the policy to adopt. This aspect of the invention will be discussed further below. Also, while the description refers to the use of four utility values, it is possible to reduce the number of inputs to generate the threshold value to a two- or even a single-parameter input system, as will described in greater detail below.

The utility values in the present implementation will however, refer to fixed values for greater ease of description, unless otherwise indicated.

The other major change from the telecare system discussed against Figure 2, is the inclusion of the resource forecaster (RF) (40) in the remote platform (20). The RF's primary function is to forecast an anticipated number of alarms that will be generated following the (proposed) adoption of the threshold values. This allows the CP to evaluate the expected draw on its resources based on the particular policy which is in turn based on the utility values adopted in the policy. In the present implementation, historic data from the sensor data store (24) is taken into account and the data in the records for all the nodes in the user base are combined for forecasting purposes. The historic data can be taken from data of the entire user base, or alternatively could pertain only to the particular node at the particular time period (e.g. the node in the kitchen for the period 00:00 to 02:00). With the forecast obtained, the CP can calculate expected budgets and other resource-related requirements. The skilled person would appreciate that various additional factors could be used in this aspect of the invention: for example, anticipated changes in the size of the user base indicated by trends apparent from the data in the CP store (34) could be included. This information can then be fed into a CP terminal (12). For any CP with limited resources, this is central to the planning, set up and operation of the telecare system.

The CP terminal (12) functions to receive data input, as well as to display the resource requirements (36) forecasted by the RF component. In the embodiment where the system uses utility values which are not fixed, it also ensures agreement from the CP on this fundamental aspect of policy, prior to the adaptive thresholds being deployed by the system.

In summary, the system architecture of this implementation of the invention allows for the following advantages to be realised in the set up and operation of a telecare system:

- The CPPE engine allows for transparent policy decisions to be embedded in threshold values set by the system
- The RF component ensures alarm volumes fall within the resources of the CP
- Utility values can be formed from multiple information sources to enable the CPPE to generate thresholds aligned with the CP's chosen telecare policy
- The care provider is aware of utility values, expected outcomes, and expected alarm volumes.

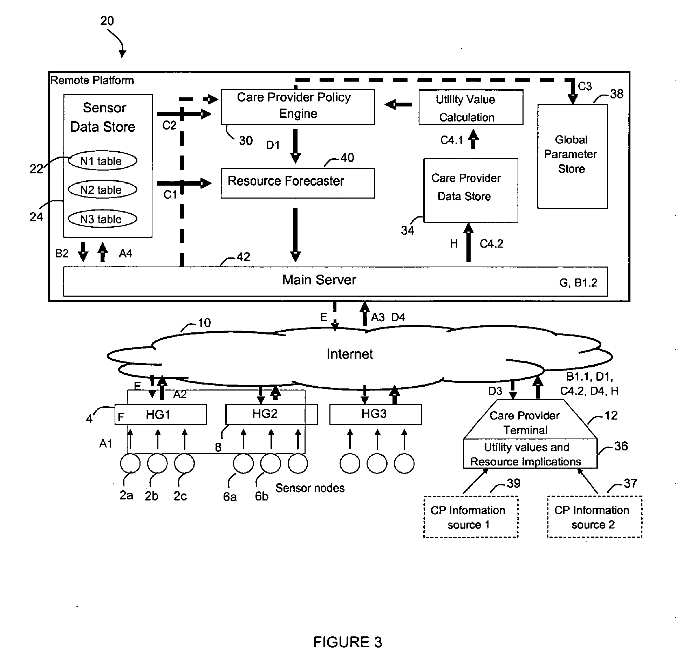

The specific processes involved in generating and using the threshold values will now be discussed in connection with the exemplary implementation of invention shown in Figure 3, the main functions and processes of which may be categorised as follows:

A    Collect and store sensor data
B    Initiate threshold setting
C    Adaptive threshold calculation
D    Forecast resource requirements
E    Global utility value setting
F    Normal operation
G    Threshold Review
H    Alarm sensitivity adjustment

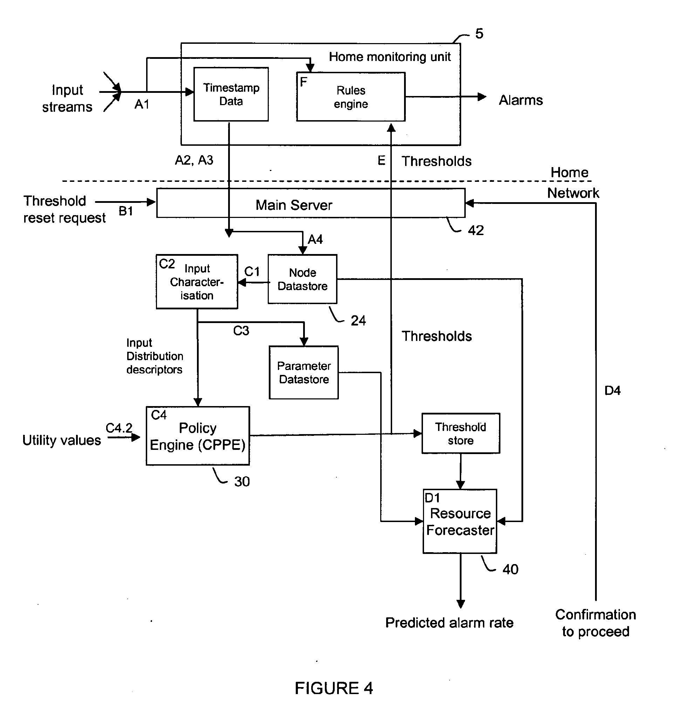

These steps are also outlined in the process flows shown in Figure 4.

*A1 - Data collection*

The process typically starts with the gathering of sensed data, as depicted by process flow arrow A1. Each sensing node (2a, 6a, etc.) is e.g. a passive infra red (PIR) sensor deployed within the home of the monitored SU detects movement within its field of view. When SU activity is detected, this fact is communicated to the home monitoring unit (5) or gateway device (4, 8 etc.) via a communications network which can be wireless (e.g. Wi-Fi, Zigbee) or a wired connection. Preferably each room in has at least one node to ensure near continuous coverage of the SU's activities as he or she moves through the premises, although the described invention could be used with fewer nodes.

*A2 - Communication with remote telecare platform*

After receiving data from the sensor nodes, the home gateway device may add a timestamp to the sensor events (if the event is not already time-stamped). The events are then forwarded e.g. over a secure Internet connection to the remote telecare platform server (20), as depicted by process flow arrow A2. In a preferred embodiment, the gateway calculates the time elapsed between sensed events, and send the inter-event time interval data to the remote platform. As data is typically sent by the nodes after each sensing, the data is preferably stored at the gateway device so that the data can be batch-processed, e.g. once a day, or else it can be passed to the remote platform in a continuous stream over the network connection (e.g. ADSL).

As the skilled person would appreciate, the processing of sensed node data to generate the thresholds according to the method of the invention is best performed at the central server platform (20) for a variety of reasons, e.g. so that the more expensive equipment required need not be replicated in each dwelling. Owing to issues relating to the resilience of the network connection between the premises and the remote platform, onsite back-up support is a preferred optional functionality provided by the home gateway. Ideally this should be a simpler method in the form of e.g. an uncomplicated rules-based algorithm to generate a simple yes/no for raising an alarm.

A preferred optional functionality of the home gateway is to provide a back-up, uninterrupted power supply in case of power loss from central sources. Another preferred option to include in the home gateway is a data buffer to prevent or reduce data loss in the absence or reduction of network connectivity.

### A3, A4 - Store data in database

The primary function of the main server (42) in the remote platform (20) is to coordinate the operations of the entire system.

Sensor data from the nodes (2a, etc.) arrives from the home gateways (4, etc.) via the Internet as depicted by process flow arrow A3. The data is stored in tables (22) (in the form of e.g. a mySQL database) in the sensor data store (24) for future access, as depicted by process flow arrow A4. The stored data consists of at least a timestamp for each piece of sensed data and an identifier of the particular node which sent the information (2a, etc.); optional additional information could include an SU identifier, a sensor node state (e.g. battery status), and so on.

The following is an example of what (part of) such a table might look like:

Table 3 Sensor node data

| Node ID | | | |
|---|---|---|---|
| Time period | Start Time | End Time | No. of inter-event times sensed |
| 4 | 08:00 | 10:00 | 1 at 08:02 1 at 08:04 (etc.) |
| 5 | 10:00 | 00:00 | 1 at 10:02 1 at 10:04 (etc.) |

### B1 - Decision or request to set thresholds

The process now moves to the setting (and re-setting) of the threshold value for the particular node for a particular time period e.g. Time Period 4 (08:00 to 10:00). In the present embodiment, this process B1.2 is carried out by the telecare main server (42). The threshold setting or re-setting process can be initiated in a number of ways. For example:

- The threshold establishment or re-set process could be automatically triggered when the number of sensed events by a particular sensor node reaches a pre-set number (e.g. 100 incidences of sensed data indicating SU activity) either for the first time or since the threshold for that node was last set or re-set.
- The process can also be automatically initiated upon at pre-set periods or upon a specific time period elapsing, e.g. once a month, or upon the passage of a month since the last threshold setting or reset.
- Alternatively, a specific request can be made manually made by the CP via the care provider terminal device (12), e.g. by selecting such an option on a web portal, as indicated by process flow arrow B1.1.

### B2 - Sample size check

Upon initiation of the process to obtain a new or an updated threshold value, the main server (42) starts by checking that a sufficient sensed data exists for that node in the sensor data store (24), as discussed further below against step C2. A sample size that is too small will impede the generation of a statistical description metric of variations in the inter-event time interval data. The more sensed data there is to process therefore, the greater the likelihood of determining a usefully accurate threshold using the process of the invention.

As the sample data requires time and resources to gather, what is "sufficient" in the particular case depends on how representative the data is required to be of the overall distribution. In this example, 100 sensor events has been taken to be the minimum number of sensed events necessary.

In the described embodiment, the process will proceed only if this condition of sufficient sample size is met, returning an exception if it is not. Alternatively, the system can present the CP or other user with the option of whether to proceed or not. In an exceptional case to proceed even without sufficient sample data (e.g. in the case of a first threshold setting), pre-existing data from pre-service trials of the system or in the form of simulated or historical data based on test installations obtained from that or other nodes, may be used.

_C1 - Read in relevant data from sensor data store_

The relevant data used for processing takes the form of inter-event times i.e. when no activity is sensed, or the time elapsed between positive sensed events. References to "data", "sensed data" and the like include references to such "negative events". This data is retrieved by the CPPE (30) from the sensor data store (24) as shown by process step arrow C1.

SU behaviour varies according to times of the day: for example, a node located in the bedroom is more likely to sense activity during the time when the SU is in the room, while the kitchen will be occupied during mealtimes. Conversely the front door is unlikely to be moved in the early hours. To increase the accuracy of assessment of the type of sensed data, this may be classified into different time periods and distribution fitting (discussed further below against process step C2) applied to each time period $\tau$. In the example discussed, the day is divided into two-hour time periods in the following way, although the skilled person would appreciate that other period divisions are possible with implications for the granularity of the assessment of the relevant data.

Table 4 Sensor node time periods

| Time period | Start Time | End Time |
|---|---|---|
| 0 | 00:00 | 02:00 |
| 1 | 02:00 | 04:00 |
| 2 | 04:00 | 06:00 |
| 3 | 06:00 | 08:00 |
| 4 | 08:00 | 10:00 |
| 5 | 10:00 | 12:00 |
| 6 | 12:00 | 14:00 |
| 7 | 14:00 | 16:00 |
| 8 | 16:00 | 18:00 |
| 9 | 18:00 | 20:00 |
| 10 | 20:00 | 22:00 |
| 11 | 22:00 | 24:00 |

In addition to dividing the data into time periods the inter-event time data is also divided according to the particular sensor which obtained the particular piece of sensed data. Statistical fitting is therefore carried out for each sensor and each time period.

To avoid having to fit the data for a large number of sensors, the data can be classified in a variety of meaningful ways. For example, it is within the scope of the invention to treat data as being representative of a certain type or class of node (e.g. they all door opening/closing sensors, etc.). Another way is to categorise the data according to the location of the node, so that In other words, if a single dwelling has more than one room of a particular class then these are all considered to be the same room type.

In the specific embodiment discussed here, data was not assigned to the classes of types of nodes, but was assigned to nodes located in the following classes of rooms in the premises, for example, the bedroom, the lounge, the bathroom/toilet, the kitchen, the hall/landing, and so on.

_C2 - Fit probability distribution to data_

For the CPPE (30) to generate the thresholds using the utility theory approach described above, the data is initially fitted to a probability distribution. This preliminary step may be thought of simplistically as "determining what normal looks like". Outlier data may be disregarded during from the process, to ensure the distribution is not excessively influenced by atypical values. A simple way to achieve this is to disregard a few of the highest and lowest values, e.g. the top and bottom 2% of data.

A variety of different statistical distributions can be fitted to the data. The example used for discussion purposes is the Weibull distribution, which has a probability density function of the form:

$$f(t) = \frac{\beta}{\eta}\left(\frac{t}{\eta}\right)^{\beta-1} \exp\left\{-\left(\frac{t}{\eta}\right)^{\beta}\right\} \qquad \text{Equation 1}$$

$\beta$ is known as the "shape parameter" and $\eta$ is known as the "scale parameter".

The cumulative distribution function is therefore given by:

$$F(t) = 1 - \exp\left\{-\left(\frac{t}{\eta}\right)^{\beta}\right\} \qquad \text{Equation 2}$$

Having selected the chosen distribution function, there are also a number of different ways of fitting the distribution to the data. In the example discussed, a standard Bayesian Regression algorithm has been used, although other methods such as Maximum Likelihood or the Method of Least Squares can be used. As noted above against step B2, an adequate representative sample size is required to obtain an accurate fit: in the current embodiment a sample size of 100 values was deemed sufficient for the purpose.

Figure 4 depicts an example of a Weibull distribution fitted to inter-event time data gathered when the SU was present in the room in question. The x-axis of the graph represents the time duration of the inter-event time period (i.e. the length of time between sensed SU activity), while the y-axis represents the probability that the SU is active in the room being monitored - and is therefore well and not requiring assistance. Thus, the longer the period of inactivity, the more likely it is that the SU needs help for which an alarm should be raised.

The key output from this step in the system operation is the two Weibull shape and scale parameter values.

*C3 - Store Fitting Parameters*

The fitting parameters obtained above are stored in the global parameter store (38) as depicted by process flow arrow C3. Each node will therefore have a minimum of two parameters per sensor node. In the present example, the global parameter store contains two fitting parameters per period per room class per SU.

*C4 - Calculate threshold based on utility values*

Once the Weibull parameter values $\beta$ and $\eta$ have been calculated, the threshold value can be calculated for each time period using the two expected utility equations below, Equations 3 and 4. These are non-standard equations derived by the applicants.

The utility values $U_0$ $U_1$ $U_2$ and $U_3$ may either be static or fixed values and are obtained (as shown in process flow arrow C4.2) from the current utility value table in the CP data store (34). Alternatively, they may be calculated from a function of different input types held in the CP data store, as will be discussed further below. As another alternative, the utility data input may be requested from the CP terminal (12) via the Internet as depicted by process flow arrow C4.2. As noted above, use of these utility values provides a transparent view of the CP's telecare policy and preference for certain decisions and outcomes over others, and makes clear the implications of the utility values adopted.

Put in general terms, the expected utility of doing nothing after observing no movement for an inter-event time period $\tau$, i.e. EU($D_0$) is given by the probability, $p(H_0|t > \tau)$, of the SU being fine and not needing help, given that the interval has now exceeded some time, $\tau$, multiplied by the utility ($U_1$) of not responding when the SU is fine, plus the probability $p(H_1|t > \tau)$ of the SU not being fine, given that the interval has now exceeded $\tau$ multiplied by the utility of the SU not being fine when no response is made ($U_3$). This can be expressed mathematically as:

$$EU(D_0) = p(H_0 \mid t > \tau) \cdot U_1 + p(H_1 \mid t > \tau) \cdot U_3$$

The quantity $p(H_0 \mid t > \tau)$ can, according to Bayes' Theorem, be obtained by revising the initial probability $p(H_0)$ that the SU is fine in the light of the information that no positive events have been detected for the time period $\tau$ thus:

$$p(H_0 \mid t > \tau) = \frac{p(H_0) \cdot p(t > \tau \mid H_0)}{p(t > \tau)}$$

$p(H_0)$ may be referred to as the *prior* probability that the SU is fine, and is a known input, derived from known information e.g. it is widely reported that about 1 in 3 people over 65 suffer a fall per year. This can be translated into an estimate of a fall in the 2-hour time periods being considered (shown in Table 3).

$p(t > \tau \mid H_0)$, the probability of the SU not moving for a time period $\tau$ when they are in fact fine, is also known to they system, as it has previously fitted a probability distribution to the inter-movement event times in the step indicated by process flow arrow C2. This distribution is integrated by the system, from $\tau$ to infinity, by applying a standard statistical technique.

The unconditional probability $p(t > \tau)$ means the probability that the interval has reached or exceeded some time, $\tau$, and in the present case we assume that this can either happen because the user is fine AND nonetheless we have (perhaps unusually if $\tau$ is relatively large) exceeded this interval OR because the user is not fine. What this means is that the total unconditional probability of the interval exceeding $\tau$, $p(t > \tau)$, is equal to the probability of the user being fine multiplied by the probability of the interval exceeding $\tau$ even though the user is fine plus the probability of the user being NOT fine multiplied by the probability of the interval exceeding $\tau$ when the user is not fine. This can be expressed mathematically as:

$$p(t > \tau) = p(H_0) \cdot p(t > \tau \mid H_0) + p(H_1) \cdot p(t > \tau \mid H_1)$$

Clearly, since the user has to be either fine or NOT fine, $p(H_1)$ equals $1 - p(H_0)$; furthermore, we assume that the probability of any interval $\tau$ being exceeded if the user is unwell is 1 - i.e. if the user is NOT fine then there will be no further sensor recordings and a rescue will have to be carried out at some point. This gives:

$$p(t > \tau) = p(H_0) \cdot p(t > \tau \mid H_0) + \left(1 - p(H_0)\right) \cdot 1$$

Thus the first part of the sum giving the expected utility of doing nothing can be re-expressed thus:

$$p(H_0 \mid t > \tau) \cdot U_1 = \frac{p(H_0) \cdot p(t > \tau \mid H_0) \cdot U_1}{p(H_0) \cdot p(t > \tau \mid H_0) + \left(1 - p(H_0)\right) \cdot 1}$$

Similarly, $p(H_1 \mid t > \tau)$ can be re-expressed according to Bayes' Theorem thus:

$$p(H_1 \mid t > \tau) = \frac{p(H_1) \cdot p(t > \tau \mid H_1)}{p(t > \tau)}$$

Therefore, since it was noted above that $p(H_1)$ equals $(1 - p(H_0))$ and that $p(t > \tau\, H_1) = 1$, the second part of the summation for the expected utility of doing nothing can be re-expressed thus:

$$p(H_1 \mid t > \tau) \cdot U_3 = \frac{(1 - p(H_0)) \cdot 1}{p(t > \tau)} \cdot U_3 = \frac{(1 - p(H_0)) \cdot U_3}{p(H_0) \cdot p(t > \tau \mid H_0) + (1 - p(H_0))}$$

A similar analysis can be performed in respect of the expected utility of doing something. After a Weibull distribution is fitted to the inter-event times, for the specific embodiment discussed here, the probability, $p(t > \tau|H_0)$, of the interval $\tau$ having been exceeded despite the user being fine can be expressed as $\exp\left\{-\left(\dfrac{\tau}{\eta}\right)^{\beta}\right\}$ and so finally, the expected utility (*EU*) formulas for the two decisions $D_0$ (Do Nothing) and $D_1$ (respond) can be expressed as:

$$\text{EU}(D_0) = \frac{U_1 p(H_0)\exp\left\{-\left(\dfrac{\tau}{\eta}\right)^{\beta}\right\} + U_3\{1 - p(H_0)\}}{p(H_0)\exp\left\{-\left(\dfrac{\tau}{\eta}\right)^{\beta}\right\} + \{1 - p(H_0)\}} \qquad \text{Equation 3}$$

$$\text{EU}(D_1) = \frac{U_0 p(H_0)\exp\left\{-\left(\dfrac{\tau}{\eta}\right)^{\beta}\right\} + U_2\{1 - p(H_0)\}}{p(H_0)\exp\left\{-\left(\dfrac{\tau}{\eta}\right)^{\beta}\right\} + \{1 - p(H_0)\}} \qquad \text{Equation 4}$$

The above Equations 3 and 4 show how the system compares the two situations: the case where some response action is to be taken, and the case where it is not. Both of these outcomes are uncertain, and are compared using their expected utilities *EU*.

As a second example, if the distribution of inter-event times is considered as being log normally distributed; that is, the logarithms of the observed inter-event times are normally (i.e. Gaussian) distributed with known mean $\mu$ and known variance $\sigma^2$, i.e. $\log t' \sim N(\mu, \sigma^2)$

Note any non-standard normal is easily converted to the standard normal (with mean zero and variance unity) by the transformation $t \leftarrow \dfrac{t' - \mu}{\sigma}$.

The probability density function and the cumulative distribution function of the standard normal are both tabulated in statistical tables, known from e.g. Lindley and Scott "New Cambridge Statistical Tables". Computation of the probability density function $\dfrac{1}{\sqrt{2\pi}}\exp\left(-\dfrac{t^2}{2}\right)$ is trivial. The cumulative distribution function $\Phi(\tau) = \dfrac{1}{\sqrt{2\pi}}\displaystyle\int_{-\infty}^{\tau} e^{-\frac{t^2}{2}}\,dt$, however, cannot be expressed in closed form but efficient numerical algorithms could be used to evaluate it, as described in e.g. Abramowitz and Stegun "Handbook of Mathematical Functions with Formulas, Graphs, and Mathematical Tables".

In the implementation of a system according to the invention, the following generalised formulae may be used:

$$p(H_0 \mid t > \tau) = p(H_0)p(t > \tau \mid H_0)/p(t > \tau)$$

becomes

$$p(H_0 \mid t > \tau) = p(H_0)\{1 - \Phi(\tau)\}/p(t > \tau)$$

and

$$p(t > \tau) \text{ becomes } p(H_0)\{1 - \Phi(\tau)\} + \{1 - p(H_0)\}$$

So the revised form of Equations 3 and 4 are:

$$\text{EU}(D_0) = p(H_0 \mid t > \tau)U_1 + (1 - p(H_0 \mid t > \tau)U_3$$

$$= \frac{p(H_0)\{1 - \Phi(\tau)\}U_1 + \Phi(\tau)U_3}{p(H_0)\{1 - \Phi(\tau)\} + \{1 - p(H_0)\}}$$

Similarly,

$$\text{EU}(D_1) = p(\dot{H}_0 \mid t > \tau)U_0 + \{1 - p(H_0 \mid t > \tau)\}U_2$$

$$= \frac{p(H_0)\{1 - \Phi(\tau)\}U_0 + \Phi(\tau)U_2}{p(H_0)\{1 - \Phi(\tau)\} + \{1 - p(H_0)\}}$$

The system then operates as before, i.e. the threshold value is set to the point where $\text{EU}(D_0) = \text{EU}(D_1)$. Note that once the threshold $\tau$ is identified it must be transformed back to the original time scale $\tau' \leftarrow \exp(\tau)$.

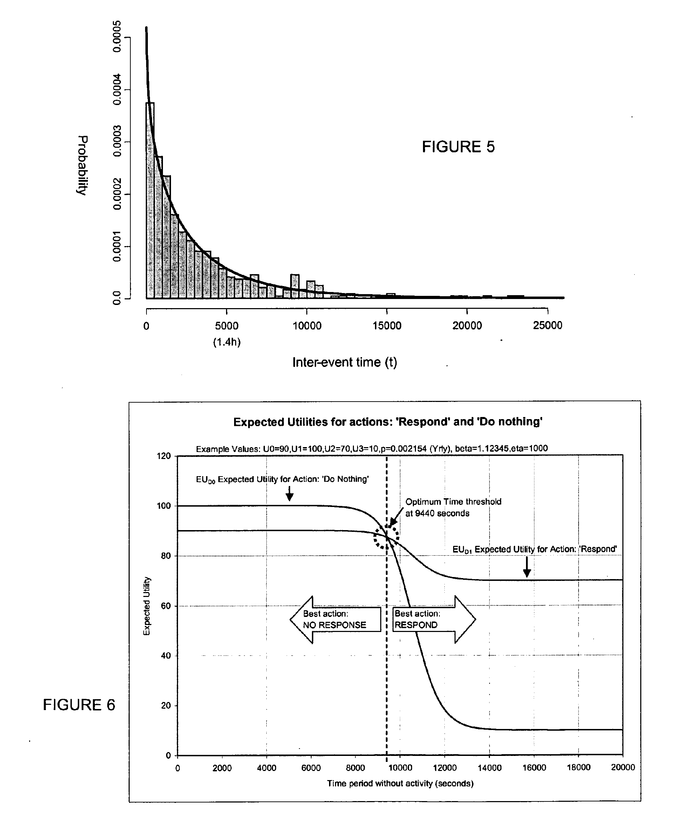

In this exemplary implementation, the appropriate threshold value for each node is arrived at by finding the inter-event time at which the two expected utilities $EU$ are equal. Figure 5 shows an example where the utility curves corresponding to the Equations 3 and 4 respectively for $\text{EU}(D_0)$ and $\text{EU}(D_1)$ have been plotted with example utility values chosen solely for illustrative purposes.

For the example values used, the optimum time at which to respond is at or after 9440 seconds (about 2.62 hours) of no sensed SU activity as illustrated by the crossover point in the graph of Figure 5, i.e. where $\text{EU}(D_0) = \text{EU}(D_1)$. At this point, the assumption is that the node's failure to sense a positive event within the time (t) elapsed between sensed events is an indication that something has probably gone wrong, so that an alarm should be raised. As can be seen from Figure 5, the choice of this time as the intervention point is dependent on the utility values chosen for $\text{EU}(D_0)$ and $\text{EU}(D_1)$ functions; if the CP had selected different utility values, a very different intervention time may be selected.

In the case where more than one "Do something" $D_1$ exists, a number of expected utility curves for e.g. $D_2$, $D_3$, $D_4$ can be generated. The skilled person would appreciate that these curves can be plotted on e.g. the graph of Figure 6, where the $\text{EU}(D_2)$ etc. curves would intersect with the $\text{EU}(D_0)$ curve at various points.

Locating the crossover point does not require the *EU* curves to be plotted on a graph as shown in Figure 5 - this can instead be achieved automatically using an algorithm such as the Bisection Method (Kronsjö, Lydia I. "Algorithms: Their Complexity and Efficiency" John Wiley and Sons (1979)). This is an iterative method for finding a real root of a equation $f(x) = 0$, where values $a$ and $b$ are known, such that $f(a)f(b) < 0$. Without loss of generality, it is assumed that $f(a) < 0$ and $f(b) > 0$. A new approximation is found by computing $(a+b)/2$ and finding the sign of $f((a+b)/2)$. If $f((a+b)/2) = 0$ the root has been found, otherwise:

$$\text{If } f\left(\frac{a+b}{2}\right) > 0 \text{ then } \frac{a+b}{2} \text{ replaces } b$$

$$\text{If } f\left(\frac{a+b}{2}\right) < 0 \text{ then } \frac{a+b}{2} \text{ replaces } a$$

The process is repeated as long as necessary. After m iterations, the root will either be found or known to lie in an interval of length $|b - a|/2^m$. The midpoint of this interval may be taken as an estimate of the sought value, having a maximum error of $\frac{1}{2}|b-a|/2^m$.

In another exemplary implementation, the solution (in the form of the intervention time value) can be assumed to occur within a 24-hour period. The function $EU(D_0) - EU(D_1)$ can be used as the $f(x)$, wherein the search space is halved until an interval is reached which is so small that it makes no substantial difference (e.g. 0.1 minutes).

A slight improvement in the accuracy of this method could be gained by the use a more sophisticated root finding algorithm, if this was required then it is preferable to use one such as Brent's algorithm (Brent, R.P. "An Algorithm with Guaranteed Convergence for Finding a Zero of a Function" Comp. J. 14,422-425) that does not require the calculation of analytic derivatives.

### D1 - Forecast alarm volumes

Having established the appropriate threshold values for all time periods, rooms and SUs, the thresholds may then be compared to historical data, preferably obtained recently, to anticipate the mean number of alerts that may be raised per week per SU whilst the system is live. In other words, knowledge of the Weibull shape and scale parameters $\beta$ and $\eta$, and the threshold values allows for a comparison may be made with historical data to assess how many times those thresholds would have been exceeded. This is a useful guide to the expected number of alerts, which is a key component of the CP's running costs. Alternatively, it is possible to calculate the expected alert rate, by integration of the probability density function from the threshold to infinity, once the distribution and the threshold value is known.

The mean expected number of alerts may then be used to predict the alarm volume for a time period e.g. a calendar quarter, or the remainder of the financial year. This value could be returned either as a total number of alarms over a period or, if the average cost per alarm was also entered by the CP, this could generate a forecasted budgetary requirement. This is of course purely an initial estimate, as factors like service user churn will affect the accuracy of the value obtained.

### D2 - Forecast changes in user base

Optionally, the system could be set up to forecast the likely changes in the size of the user base over time by using standard statistical approaches. See for example, Chris Chatfield "The Analysis of Time Series" (CRC/Chapman and Hall).

### D3 - Forecast Request Confirmation

The result obtained through the process of D1 or D2 is made available to the CP via the CP terminal (12). Once the CP confirms acceptance of the forecasted figures, the system can proceed to the next step, i.e. with the threshold updates. Confirmation may be automatically generated, e.g. where forecasted values stay within allowed pre-set bounds.

In the current embodiment, fixed utility values are used for simplicity to describe the system and in many situations will be the preferred mode of operation of the invention. Use of fixed values requires only one confirmation of acceptance from the CP. However, if the utility values are not fixed, but generated by a function as explained in step C4.1, then these may also be confirmed by the CP at this stage.

*D4 - Confirmation*

The CP's agreement to forecasted alarm volumes and any altered utility values are then communicated to the main server (42).

*E - Set global utility values*

Once the CP has selected and accepted the utility values and the alarm volumes associated therewith, the utility values can be set globally across the SU base, or where relevant, within segments of the SU base. These may be set as initial base values to ensure that personalised thresholds are provided across the entire base so that SUs within the base or segment are treated equitably. Preferably, these base values can be subsequently adjusted as required e.g. upon an SU's request to reduce or enhance alarm sensitivity as described further below.

*F - Normal operation / Threshold enforcement*

In the present exemplary embodiment, appropriate threshold values for a particular time period, room and SU are also downloaded to the gateway device of the relevant node and SU. This creates a secondary alarm system based on the locally-stored data, which would provide a measure of resilience of operation. For example, in the case of loss of standard network (e.g. ADSL) connectivity between the remote telecare platform (20) and an individual gateway device (4, 8, etc.), a connection could still be created using e.g. GPRS. The threshold checking functionality could however also be provided by the main server platform (42) if the gateway devices (4, 8) continually streamed data to the server platform.

Once the threshold value has been set for the node, the system is said to be operating "normally" when its task is simply to sense inter-event times and to report these back to the main server. When these times exceed the prescribed threshold, the system goes into a "threshold enforcement" state, wherein the gateway device determined the existence of an abnormal time interval between events to e.g. the CP terminal (12), when such an exception occurs. It is also possible for the gateway to simply communicate sensed events to the telecare main server, and for the main server to perform the task of calculating that the time elapsed between events has exceeded the threshold value of the particular sensing node.

*G - Threshold resets*

During normal operation there may or may not be inter-event threshold value resets (along the lines of the process described in section B1 above) and these may or may not require the service provider to sign off or confirm its approval of utility values and alarm volumes (as described against sections B1 and D above).

*H - Alarm sensitivity adjustment*

It is possible that an SU (or other party) may request an adjustment to the alarm sensitivity, e.g. due to the perceived nuisance of a seemingly-oversensitive system. Such an SU may choose to assume more risk in order have fewer false alarms generated. In a preferred implementation, the system may be configured to provide to the CP the flexibility to so tweak and adjust the operation of mot just the threshold and/or alarm settings, but any part of the system - if the CP's adopted policy allows for this. In practice, changes made to the system may affect all across the SU base, although it is possible to adjust the sensitivity of the system of just the requesting SU.

Even in a case where all the settings automatically derived by the system were subsequently manually over-ridden, the utility theory based system still offers advantages in generating an empirically-derived baseline value. This makes the process of selecting (or adjusting) an individual's utility values transparent to both the SU, the CP and any other interested party.

If this functionality allowing manual adjustment is provided by a CP, then the system maintains a table of "customised utility values" (separate from the table of utility values) which are system-generated or otherwise derived. Both types of tables can be maintained in the CP data store (34), and the data therein can be called upon when setting or adjusting threshold values e.g. for those SU or other parties who request or require more, or less, alarm sensitivity from the system.

*Application examples*

The previous section describes the main architecture and process flows of a basic implementation of the invention. The following will outline details of two potential applications of a system of the invention, to show its flexibility in deployment and how it can be tailored for use in different situations and to deliver different functionalities.

*1. Multiple SU attendance*

In the typical situation, when a CP receives an alarm regarding a particular SU, this alarm is shown on the CP's terminal and the relevant SU may be identified as having a "red status" i.e. is in need of immediate assistance. The SU requires an immediate home visit and dispatch a care worker to the SU's premises. After dealing with the situation the care worker would typically return to base. The action of dispatching a care worker represents a cost to the CP, of which a significant proportion is attributable to travel time and other resource consumed by travelling to the SU's premises.

In a preferred embodiment of the invention, the system can also identify "amber status" SUs who are geographically located near to the "red status" SU. Such "amber status" SUs are those individuals whose sensed inter-event times have not exceeded the prescribed threshold values, but whose data is nonetheless giving cause for concern e.g. the times are very near the threshold points. The location of SUs within the system can be identified by providing that one of the CP information sources (37, 39) providing input to the CP terminal (12) comprises geographic location information of SUs.

On raising the "red status" alarm the system may now also identify one or more "amber status" SUs who are within e.g. 5 miles of the "red status" SU, or who may be located on the care worker's route to or from the "red status" SU. This is performed by including an "amber" utility value calculation using a function incorporating a geographic proximity factor. The set of utility values passed to the CPPE (30) will reflect the cost-efficiency (i.e. increased utility) in visiting such an "amber status" SU when going out to attend to the "red status" SU. This reduces the per-individual cost of attending to a number of SUs - and advantageously may catch a cause for concern before the situation develops to a "red status", thus possible preventing another "red status" alarm which will have to be attended to separately at full cost.

From the CP's perspective, the system facilitates more timely and cost-efficient intervention, while from the SUs' perspective they will benefit from more, and more frequent visits by a care worker at times when the sensed data indicates that they may be on the verge of needing help. This provides them with increased social contact and a superior service.

In a further implementation of the invention, the system could prioritise "amber status" SUs to order the visits in dependence on need (e.g. the closeness of the sensed inter-event time data to the threshold value in each case). This could be performed by allocating "shades of amber" for all SUs who are geographically related to the "red status" SU during the call-out.

In practice, the cost/utility of sending a care worker to attend to an SU is seldom a function of a single variable. Factors affecting the travel time and conditions, the availability and qualification of the care worker, the actual condition of the SU, are just some of the influences on the duration and complexity of the call out and the overall cost. The system permits the CP to include estimates of such parameters by adding these to the CP information sources (37 and/or 39), which data can be retrieved via the CP terminal (12) as shown by process arrow 4.2 in Figure 3. The utility value calculation is then performed on this richer, more complex data comprising multiple inputs, which makes the system well suited to a holistic approach to the CP's planning and management activities.

For example, a telecare call centre can have on call only a few highly-skilled care workers out of all the other members of staff. By also including this factor in the "amber status" utility value calculation, the few highly-skilled care workers could be shared between several call centres, making for greater cost-efficiency in staffing and operation.

*2. Recipients of alarms*

Within a single system, alarms may be directed to more than one destination in dependence e.g. on the identity of the SU in question. The destination or routing for a particular alarm could be assigned based on the SU's medical conditions which are already known. For example, patients who have already been assigned a particular care worker (perhaps one with particular expertise in the particular medical condition) may have their alarms always routed to that care worker. SUs without a pre-assigned care worker may, on the other hand, have their alarms routed to a central telecare call centre. In such a way, the invention acts in part as a router, aimed at maximising the efficiency and utilisation of the call centre and care workers, and thus helping to reduce overall operating costs.

The skilled person would appreciate that yet further implementations and uses can be made of and within a remote monitoring system arranged and operated according to the invention.

The above exemplary methods and apparatus described can also be used or "retrofitted" to a conventional telecare or other remote monitoring system which includes model fitting capability.

The methods and configurations as described above and in the drawings are for ease of description only and not meant to restrict the apparatus or methods to a particular arrangement or process in use. In particular, the methods and apparatus described are merely exemplary and may be usefully deployed in any remote monitoring system, such as the condition of factory plant and machinery, parts of consumer goods, cars or the like, and so on. It would be appreciated that the receipt of abnormal or unexpected values in the telecare context could also result from malfunctioning components within the telecare system, e.g. where the nodes, gateway devices, and the like, are not working as they should.

It will be apparent to the skilled person that various sequences and permutations on the methods and apparatus described are possible within the scope of this invention as disclosed.

**Claims**

1.  Apparatus for alerting an operator to the status of a monitored system, the apparatus comprising:

    - input means for permitting an operator to input utility values associated by the operator with different courses of action and different states of the system being monitored, such that each utility value expresses the perceived value to the operator of carrying out different courses of action when the monitored system is in each of a plurality of different states;
    - monitoring means for estimating the probability of the monitored system being in each of a plurality of different states based on sensed data;
    - expected utility calculation means for calculating an expected utility for each possible course of action based on the utility values associated with the different courses of action and the estimated probabilities of the different possible states of the monitored system; and
    - alerting means for alerting the operator if a predetermined condition based on the calculated expected utilities is satisfied.

2.  Apparatus according to claim 1 wherein the predetermined condition comprises the situation wherein the expected utility for two possible courses of action are equal.

3.  Apparatus according to claim 1 or claim 2 wherein the monitoring means comprises a statistical engine arranged to fit historically sensed data to a probability distribution for subsequent use by the monitoring means together with current sensed data in order to estimate the probability of the monitored system being in each of the plurality of different states.

4.  Apparatus according to claim 3 wherein the statistical engine is arranged to fit the historically sensed data to a Weibull probability distribution to obtain a shape parameter and a scale parameter characterising the sensed data.

5.  Apparatus according to any preceding claim wherein the input means includes a utility value engine to calculate utility values based on input by the operator.

6.  Apparatus according to any preceding claim including a forecaster to forecast an expected number of alerts over a specified period.

7.  A remote monitoring system including

    - apparatus according to any preceding claim, and
    - a node for collecting the sensed data.

8.  A telecare monitoring system including the remote monitoring apparatus according to claim 7.

9.  A method for alerting an operator to the status of a monitored system, the apparatus comprising:

    - an operator inputting utility values associated by the operator with different courses of action and different states of the system being monitored, such that each utility value expresses the perceived value to the operator of carrying out different courses of action when the monitored system is in each of a plurality of different states;
    - estimating the probability of the monitored system being in each of a plurality of different states based on sensed data;

- calculating an expected utility for each possible course of action based on the utility values associated with the different courses of action and the estimated probabilities of the different possible states of the monitored system; and
- alerting the operator if a predetermined condition based on the calculated expected utilities is satisfied.

10. A method according to claim 9 wherein the predetermined condition comprises the situation wherein the expected utility for two possible courses of action are equal.

11. A method according to claim 9 or claim 10 wherein the step of estimating the probability comprises fitting historically sensed data to a probability distribution for subsequent use by the monitoring means together with current sensed data in order to estimate the probability of the monitored system being in each of the plurality of different states.

12. A method according to claim 11 wherein the step of estimating the probability comprises fitting the historically sensed data to a Weibull probability distribution to obtain a shape parameter and a scale parameter characterising the sensed data.

13. A method according to any one of claim 9 to 12 further including a step of forecasting an expected number of alerts over a specified period.

14. A method of operating a remote monitoring system including

   - collecting sensed data, and
   - the steps of a method according to any one of claims 9 to 15.

15. A method of operating a telecare monitoring system using the method of operating the remote monitoring apparatus according to claim 14.

20

Remote Platform

Sensor
Data

23

N1 table — 22

N2 table

N3 table

ATA calculation

Arbitrary fixed value

Personalised thresholds

10 — Internet

HG1

HG2

HG3

Care Provider Terminal — 12

4

8

2a  2b  2c

6a  6b

Arbitrary value

FIGURE 1

FIGURE 2

FIGURE 3

EP 2 098 970 A1

FIGURE 4

22

FIGURE 5

Inter-event time (t)

FIGURE 6

**European Patent**
**Office**

**DECLARATION**

which under Rule 63 of the European Patent Convention
shall be considered, for the purposes of subsequent
proceedings, as the European search report

Application Number

EP 08 25 0773

| The Search Division considers that the present application, does not comply with the provisions of the EPC to such an extent that it is not possible to carry out a meaningful search into the state of the art on the basis of all claims | CLASSIFICATION OF THE APPLICATION (IPC) |
|---|---|
| Reason:<br><br>The present application contains 6 independent claims (1,7,8,9,14,15). There is no clear distinction between the independent claims because of overlapping scope. Moreover, the present set of claims, when considered as a whole, is drafted in such a way that the claims as a whole are not in compliance with the provisions of clarity and conciseness of Article 84 EPC (the wording of the claim is too broad and vague), as it is particularly burdensome for a skilled person to establish the subject matter for which protection is sought. The non-compliance with the substantive provisions is to such an extent, that no meaningful search was considered as possible.<br><br>The applicant's attention is drawn to the fact that a search may be carried out during examination following a declaration of no search under Rule 63 EPC, should the problems which led to the declaration being issued be overcome (see EPC Guideline C-VI, 8.2).<br>----- | INV.<br>G06F19/00 |

| Place of search | Date | Examiner |
|---|---|---|
| Munich | 7 August 2008 | Samulowitz, Michael |

EPO FORM 1504 (P04F37)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 07253336 A

**Non-patent literature cited in the description**

- **Barnes et al.** Case Studies from the Liverpool Telecare Pilot. *Informatics in Primary Care,* 2006, vol. 14 (3), 197-202
- **Kronsjö, Lydia I.** Algorithms: Their Complexity and Efficiency. John Wiley and Sons, 1979
- **Brent, R.P.** An Algorithm with Guaranteed Convergence for Finding a Zero of a Function. *Comp. J.,* vol. 14, 422-425
- **Chris Chatfield.** The Analysis of Time Series. CRC/Chapman and Hall